(19) Europäisches Patentamt
European Patent Office
Office européen des brevets

(11) Veröffentlichungsnummer: **0 003 501**
**B1**

(12) # EUROPÄISCHE PATENTSCHRIFT

(21) Anmeldenummer: **79100069.8**

(22) Anmeldetag: **11.01.79**

(51) Int. Cl.³: **C 07 D 277/30, C 07 D 277/64, C 07 D 417/04**

(54) **Verfahren zur Herstellung von Thiazolderivaten**

(30) Priorität: **17.01.78 DE 2801794**

(43) Veröffentlichungstag der Anmeldung:
**22.08.79 Patentblatt 79/17**

(45) Bekanntmachung des Hinweises auf die Patenterteilung:
**07.01.81 Patentblatt 81/01**

(84) Benannte Vertragsstaaten:
**CH DE FR GB IT**

(56) Entgegenhaltungen:
**BE - A - 463 365**
**BE - A - 477 772**
**DE - A - 1 913 472**
**GB - A - 609 919**
**US - A - 2 263 018**
**US - A - 3 498 997**

**THE JOURNAL OF ORGANIC CHEMISTRY, Vol. 19, Nr. 12, Dezember 1954, Baltimore-2, USA P.L. SOUTHWICK et al.: "4-Styrylthiazoles. Syntheses and relationships among ultraviolet absorption spectra", Seiten 1926—1937**

**SITZUNGSBERICHTE, Abteilung IIb, Chemie, 162. Band, Heft 3—4, 15. April 1953, Wien R. Riemschneider et al.: "Thiocarbamete. Mitt. VII: Einwirkung von konz. Schwefelsäure auf aliphatische und hydroaromatische Rhodanide", Seiten 313—318**

(73) Patentinhaber: **BASF Aktiengesellschaft**
**Carl-Bosch-Strasse 38**
**D - 6700 Ludwigshafen (DE)**

(72) Erfinder: **Seybold, Guenther, Dr.**
**Otto-Dill-Strasse 32**
**D - 6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 003 501 B1

Verfahren zur Herstellung von Thiazolderivaten

Die Erfindung betrifft ein Verfahren zur Herstellung der tautomeren Verbindungen der allgemeinen Formeln IA und IB

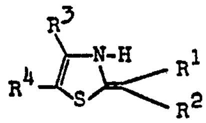 IA

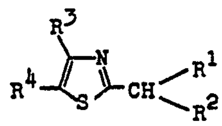 IB

in der

$R^1$ und $R^2$ Cyan, Formyl, Nitro, gegebenenfalls substituiertes Alkanoyl oder Aroyl, Carbonester, gegebenenfalls substituiertes Carbamoyl oder Sulfamoyl, Alkylsulfonyl, Arylsulfonyl oder Alkylsulfinyl,

$R^1$ und $R^2$ zusammen mit der Methin gruppe den Rest einer methylenaktiven cyclischen Verbindung,

$R^2$ darüber hinaus Aryl oder Hetaryl,

$R^3$ und $R^4$ Wasserstoff, gegebenenfalls substituiertes Alkyl, Aryl, Hetaryl, Carbonester, Alkanoyl oder Aroyl und

$R^3$ und $R^4$ zusammen einen cyclischen Rest bedeuten, dadurch gekennzeichnet, daß man Verbindungen der Formel II

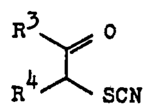 II

mit Verbindungen der Formel III

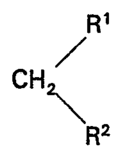 III

in Gegenwart von Basen miteinander umsetzt.

Reste $R^1$ und $R^2$ sind beispielsweise $C_1$- bis $C_8$-Alkanoyl, das z.B. noch durch Chlor, Brom oder $C_1$- bis $C_4$-Alkoxy substituiert sein kann, gegebenenfalls durch Chlor, Brom, Methyl, Äthyl, Methoxy, Äthoxy oder Cyan substituiertes Benzoyl, Naphthoyl, von $C_1$- bis $C_8$-Alkanolen, -Glykolen, -Glykoläthern oder Phenolen abgeleitete Carbonester, durch $C_1$- bis $C_8$-Alkyl, -Alkoxyalkyl, Cyclohexyl oder Phenyl substituiertes Carbamoyl oder Sulfamoyl, $C_1$- bis $C_4$-Alkylsulfonyl oder -sulfinyl, Phenylsulfonyl oder Phenylsulfinyl.

Für $R^3$ und $R^4$ sind im Rahmen der allgemeinen Definition die gleichen Reste zu nennen.

Einzelne Reste $R^1$ sind neben den bereits genannten z.B.: $COCH_3$, $COC_2H_5$, $COC_3H_7$, $COCH_2Cl$, $COCHCl_2$, $COC_6H_5$. $COC_6H_4Cl$, $COOCH_3$, $COOC_2H_5$, $COOC_3H_7$, $COOC_4H_9$, $COOC_6H_5$, $COO(CH_2)_3OCH_3$, $COO(CH_2)_3OC_2H_5$, $CONHCH_3$, $CONHC_2H_5$, $CONHC_3H_7$, $CONHC_4H_9$, $CON(CH_3)_2$, $CON(C_2H_5)_2$, $CONH_2$, $CONHC_6H_5$,

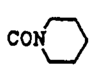 , 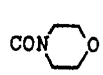 oder 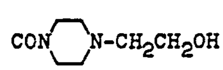

sowie die entsprechenden Sulfamoylreste, $CH_3SO_2$, $C_2H_5SO_2$, $C_4H_9SO_2$ oder $CH_3SO$;

$R^2$ kann mit $R^1$ identisch sein, daneben seien im einzelnen aufgeführt: $O_2N—C_6H_4$, $NC—C_6H_4$, $CH_3COC_6H_4$,

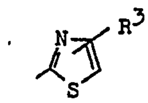, 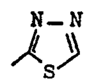, 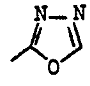,

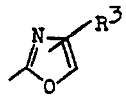, 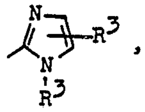, 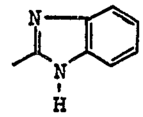,

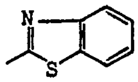, 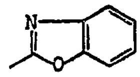 oder 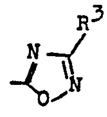.

$R^1$ kann mit $R^2$ zusammen einen cyclischen Rest bilden, z.B.:

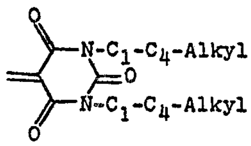, 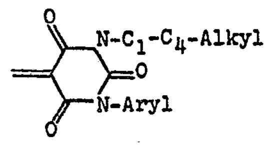,

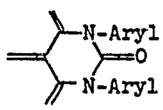, 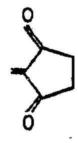, 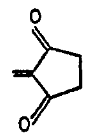,

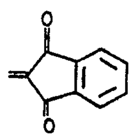, oder 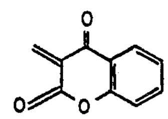

wobei Aryl gegebenenfalls z.B. durch Chlor, Brom, Methyl, Äthyl, Methoxy oder Äthoxy substituiertes Phenyl ist.

Reste $R^3$ und $R^4$ sind im einzelnen beispielsweise: $CH_3$, $C_2H_5$, $C_3H_7$, $C_4H_9$, $C_5H_{11}$, $C_6H_5$, $C_6H_{13}$, $C_7H_{15}$, $C_1$- bis $C_4$-Alkyl-$OC_6H_4$, ($C_1$—$C_4$-Alkyl)$_2OC_6H_3$, Cl—$C_6H_4Cl_2C_6H_3$, $BrC_6H_4$, $Br_2C_6H_3$, $C_1$-bis $C_4$-Alkyl-S—$C_6H_4$, $H_5C_6S$—$C_6H_4$, $C_1$- bis $C_4$-Alkyl-$CONHC_6H_4$, $COOCH_3$, $COOC_2H_5$, $COOC_3H_7$, $COOC_4H_9$, $COC_6H_5$, $COCH_3$ oder $CH_2$—COO—$C_1$- bis $C_4$-Alkyl,

$R^3$ kann mit $R^4$ einen Ring vervollständigen, z.B.

$$R^3 + R^4 = -(CH_2)_{3-6}$$

oder 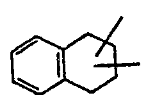

Als Basen für die Umsetzung eignen sich im Prinzip alle basisch reagierenden Verbindungen, z.B. seien Alkali- oder Erdalkaliverbindungen, Amine oder Alkoholate erwähnt.

Im einzelnen seien beispielsweise Hydroxide, wie NaOH, KOH, $Ca(OH)_2$, $Ba(OH)_2$ oder $Mg(OH)_2$, Carbonate wie $Na_2CO_3$, $K_2CO_3$ oder $Li_2CO_3$, Alkoholate wie $NaOCH_3$, $NaOC_2H_5$, $KOC(CH_3)_3$ oder $KOCH_3$ oder Amine wie $NH_3$, Äthylamin, Piperidin, Pyrrolidin, $N(CH_3)_3$, $N(C_2H_5)_3$, $N(C_4H_9)_3$, $N(CH_2CH_2OH)_3$, $C_6H_5N(CH_3)_2$ oder Pyridin gennant.

Zweckmäßigerweise wird die Umsetzung der Verbindungen II und III in einem Lösungsmittel vorgenommen. Es eigen sich z.B. Kohlenwasserstoffe, Alkohole, Glykole, Glykoläther, Amide, Äther, Ketone oder Nitrile auch im Gemisch mit Wasser.

In manchen Fällen sind auch die Basen als Lösungsmittel brauchbar.

Einzelne Lösungsmittel sind beispielsweise: Toluol, Xylole, Chlorbenzol, Methylenchlorid, Äthylenchlorid, Chloroform, Methanol, Äthanol, Isopropanol, Isobutanol, Äthylenglykol, Äthylenglykol-mono-

methyl, -äthyl- oder -butyläther, Dimethylformamid, Dimethylacetamid, N-Methylpyrrolidon, Tetrahydrofuran, Dioxan, Aceton, Methyläthylketon, Cyclohexanon, Acetonitril oder Dimethylsulfoxid.

Bevorzugt sind z.B. Methanol, Äthanol, Isobutanol oder Dimethylformamid.

Einzelheiten der Reaktionsführung können den Beispielen entnommen werden, in denen sich Angaben über Teile und Prozente, sofern nicht anders vermerkt, auf das Gewicht beziehen.

Von besonderer Bedeutung ist das erfindungsgemäße Verfahren zur Herstellung von Verbindungen der Formel I a

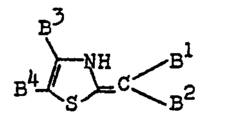

Ia,

in der

$B^1$ und $B^2$ Cyan, $C_1$- bis $C_4$-Alkoxycarbonyl, $C_1$- bis $C_4$-Alkanoyl, substituiertes Carbamoyl oder Hetaryl,

$B^3$ und $B^4$ gegebenenfalls durch Chlor, Brom, $C_1$- bis $C_4$-Alkoxy, Methyl, Acetylamino, $C_1$- bis $C_4$-Alkylmercapto substituiertes Phenyl, Methyl oder Äthyl bedeuten.

Die Verbindungen der Formel I sind wertvolle Zwischenprodukte zur Herstellung von Farbstoffen und Wirkstoffen und sind zum großen Teil neu.

Beispiel 1

4 Phenyl-thiazolylmalodinitril

33 Teile Malodinitril und 90 Teile Phenacylrhodanid werden in 500 Teilen Alkohol vorgelegt. Dann werden unter Rühren 46 Teile Triäthylamin zugetropft (Temperatur 30°C), und das Reaktionsgemisch wird über Nacht bei Raumtemperatur gerührt. Anschlußend wird mit 500 Teilen $H_2O$ verdünnt, mit Eisessig schwach sauer gestellt und das Produkt abgesaugt; feinkristallines Pulver. Ausbeute: 112 Teile ≙ 99,5%. Schmelzpunkt 255°C (Zers.).

Beispiel 2

4-Phenylthiazolylcyanessigsäuremethylester

90 Teile Phenacylrhodanid werden in ein Gemisch von 150 Teilen DMF, 150 Teilen Triäthylamin und 50 Teilen Cyanessigsäuremethylester eingetragen. Nach 4-stündigem Nachrühren bei Raumtemperatur wird in Wasser gegeben und mit Essigsäure angesäuert. Das ausgefallene Produkte wird abgesaugt und im Trockenschrank getrocknet.

Ausbeute: 129 Teile ≙ 93%, Schmelzpunkt 176°C.

Beispiel 3

4-Phenylthiazolylmalodinitril

Es wird wie unter Beispiel 1 gearbeitet, jedoch werden statt Triäthylamin 43 Teile Piperidin eingesetzt.

Ausbeute: 112 g.

Beispiel 4

4-Phenylthiazolylmalodinitril

Es wird wie unter Beispiel 1 gearbeitet, jedoch werden statt Triäthylamin 30 Teile 30%iges Ammoniak eingesetzt.

Ausbeute: 68%.

Beispiel 5

4-Phenylthiazolylmalodinitril

Es wird wie bei Beispiel 1 gearbeitet, jedoch wurden statt Triäthylamin 143 Teile Soda in wenig Wasser gelöst zugegeben.

Ausbeute: 91%.

Beispiel 6

4-Phenylthiazolylmalodinitril

Es wird wie bei Beispiel 1 bearbeitet, jedoch werden statt Alkohol 600 Teile Toluol verwendet. Das Produkt wird durch Abdestillieren des Lösungsmittels gewonnen.

Ausbeute: 46%.

Beispiel 7

4-Methylthiazolylmalodinitril

33 Teile Malodinitril werden in 400 Teilen Methanol vorgelegt; dazu gibt man 57 Teile Rhodanaceton und unter Kühlung 50 Teile Triäthylamin. Das Reaktionsgemisch wird 4 Stunden bei Raumtemperatur gerührt, in Wasser gegeben, mit Ameisensäure angesäuert und abgesaugt.

Ausbeute: 71 Teile ≙ 88%.

Beispiel 8

4-Methylthiazolylcyanessigsäuremethylester

Es werden 50 Teile Cyanessigsäuremethylester, 50 Teile Triäthylamin und 57 Teile Rhodanaceton in 400 Teilen Methanol wie bei Beispiel 7 beschrieben umgesetzt.

Ausbeute: 79 Teile ≙ 70%, Schmelzpunkt 235°C.

Beispiel 9

4-Äthyl-5-methylthiazolylmalodinitril

Es werden 14,3 Teile 1-Rhodan-diäthylketon mit 6,6 Teilen Malodinitril wie bei Beispiel 1 beschrieben umgesetzt.

Ausbeute: 12,4 Teile, Schmelzpunkt 225°C.

Beispiel 10

4-Äthoxycarbonylmethylthiazolylmalodinitril

Es werden 19 Teile $\gamma$-Rhodan-acetessigsäureäthylester mit 6,6 Teilen Malodinitril wie bei Beispiel 1 umgesetzt.

Ausbeute: 21 Teile.

Beispiel 11

Tetrahydrobenzthiazolylmalodinitril

15,4 Teile 1-Rhodancyclohexanon werden wie unter Beispiel 1 beschrieben mit 6,6 Teilen Malodinitril umgesetzt.

Ausbeute: 14,3 Teile.

Beispiel 12

Tetrahydrobenzthiazolyl-cyanessigsäuremethylester

Es wird wie bei Beispiel 11 gearbeitet, jedoch werden 10 Teile Cyanessigsäuremethylester statt Malodinitril verwendet.

Ausbeute: 16 Teile.

Die Verbindungen 13 bis 34 werden analog der für Beispiel 1 gegebenen Vorschrift hergestellt.

| Bsp. | Verbindung | Ausbeute |
|---|---|---|
| 13 | 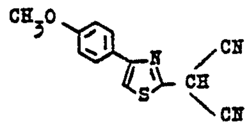 | 85 % |
| 14 | 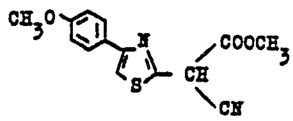 | 83 % |
| 15 | 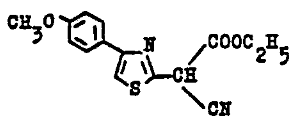 | 80 % |
| 16 | 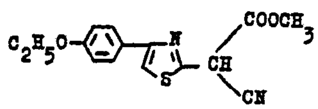 | 95 % |
| 17 | 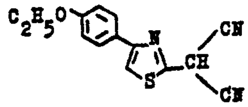 | 93 % |
| 18 | 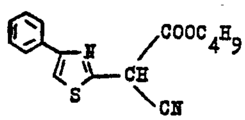 | 70 % |
| 19 | 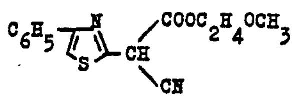 | 73 % |
| 20 | 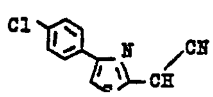 | 89 % |

| Bsp. | Verbindung | Ausbeute |
|---|---|---|
| 21 | 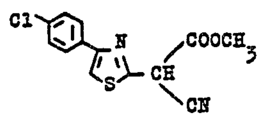 | 86 % |
| 22 | 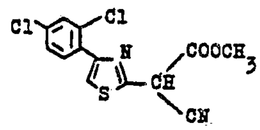 | 80 % |
| 23 | 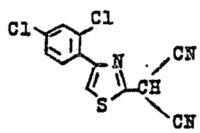 | 83 % |
| 24 | 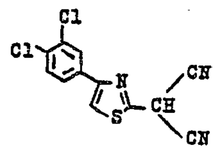 | 82 % |
| 25 | 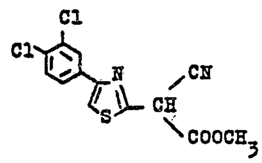 | 83 % |
| 26 | 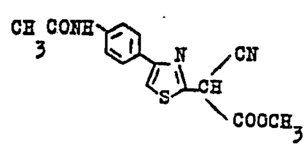 | 90 % |
| 27 | 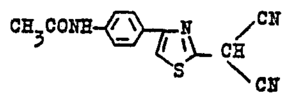 | 95 % |
| 28 | 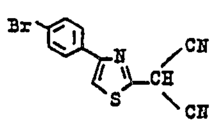 | 84 % |

| Bsp. | Verbindung | Ausbeute |
|---|---|---|
| 29 | 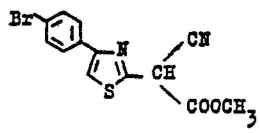 | 83 % |
| 30 | 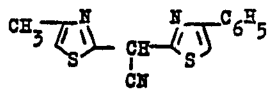 | 60 % |
| 31 | 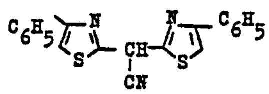 | 55 % |
| 32 | 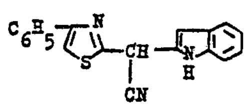 | 51 % |
| 33 | 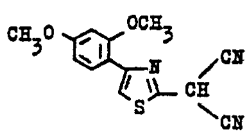 | 80 % |
| 34 | 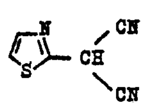 | 60 % |

Beispiel 35

18 Teile Phenacylrhodanid, 10 Teile Triäthylamin und 16 Teile N,N-Dimethylbarbitursäure werden in 100 Teilen Propanol 2 Stunden unter Rückfluß gekocht. Anschließend wird abgekühlt und das Produkt abgesaugt. Man erhält 10 Teile der Verbindung der Formel

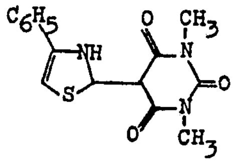

Analog werden die Verbindungen 36 bis 38 hergestellt.

| Bsp. | Verbindung |
|---|---|
| 36 | 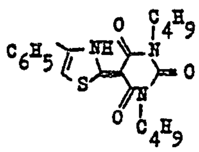 |
| 37 | 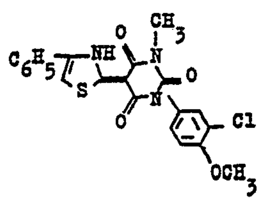 |
| 38 | 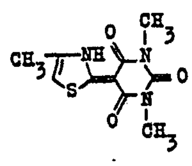 |

## Patentansprüche

1. Verfahren zur Herstellung von Thiazolderivaten der allgemeinen Formeln

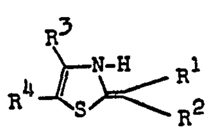 IA

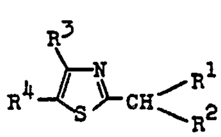 IB,

in der

$R^1$ und $R^2$ Cyan, Formyl, Nitro, ggf. substituiertes Alkanoyl oder Aroyl, Carbonester, ggf, substituiertes Carbamoyl oder Sulfamoyl, Alkylsulfonyl, Arylsulfonyl oder Alkylsulfinyl,

$R^1$ und $R^2$ zusammen mit der Methingruppe den Rest einer methylenaktiven cyclischen Verbindung,

$R^2$ darüber hinaus Aryl oder Hetaryl,

$R^3$ und $R^4$ Wasserstoff, ggf. substituiertes Alkyl, Aryl, Hetaryl, Carbonester, Alkanoyl oder Aroyl und

$R^3$ und $R^4$ zusammen einen cyclischen Rest bedeuten, *dadurch gekennzeichnet*, daß man Verbindungen der Formel II

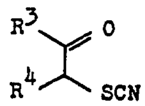

II

mit Verbindungen der Formel III

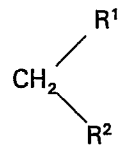

III

in Gegenwart von Basen miteinander umsetzt.

2. Verfahren gemäß Anspruch 1, *dadurch gekennzeichnet*, daß man Verbindungen der Formel

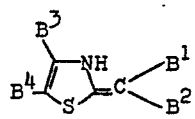

Ia

herstellt, in der

$B^1$ und $B^2$ Cyan, $C_1$- bis $C_4$-Alkoxycarbonyl, $C_1$- bis $C_4$-Alkanoyl, substituiertes Carbamoyl oder Hetaryl und

$B^3$ und $B^4$ gegebenenfalls durch Chlor, Brom, $C_1$- bis $C_4$-Alkoxy, Methyl, Acetylamino, $C_1$- bis $C_4$-Alkylmercapto substituiertes Phenyl, Methyl oder Äthyl bedeuten.

**Revendications**

1. Procédé pour la préparation de dérivés du thiazole de formules générales

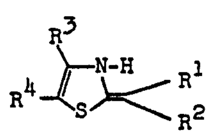

IA

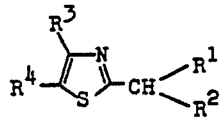

IB,

dans lesquelles

$R^1$ et $R^2$ représentent chacun un cyano, formyle, nitro, alcanoyle ou aroyle éventuellement substitué, ester carboxylique, carbamoyle ou sulfamoyle éventuellement substitué, alcoylsulfonyle, arylsulfonyle ou alcoylsulfinyle,

$R^1$ et $R^2$ forment ensemble, avec le groupe méthine, le radical d'un composé cyclique méthylène-actif,

$R^2$ peut être en outre un aryle ou un hétaryle,

$R^3$ et $R^4$ représentent chacun un hydrogène, un alcoyle éventuellement substitué, un aryle, un hétaryle, un ester carboxylique, un alcanoyle ou un aroyle et

$R^3$ et $R^4$ forment ensemble un radical cyclique, caractérisé en ce qu'on fait réagir entre eux des composés de formule II

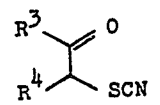

II

et des composés de formule III

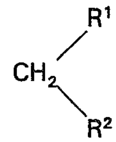

III

en présence de bases.

2. Procédé selon la revendication 1, caractérisé en ce qu'on prépare des composés de formule

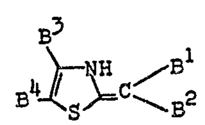

Ia

dans laquelle

$B^1$ et $B^2$ représentent chacun un cyano, un alcoxycarbonyle à 1—4C, und alcanoyle à 1—4 C, un carbamoyle substitué ou un hétaryle, et

$B^3$ et $B^4$ représentent chacun un phényle éventuellement substitué par un chlore, par un brome, par un alcoxy à 1—4 C, par un méthyle, par un acétylamino, par un alcoylmercapto à 1—4 C; un méthyle ou un éthyle.

**Claims**

1. A process for the preparation of a thiazole derivative of the general formula

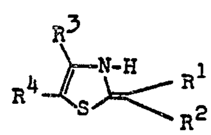

IA

or

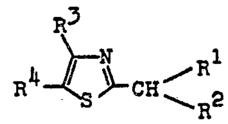

IB

where

$R^1$ and $R^2$ are cyano, formyl, nitro, unsubstituted or substituted alkanoyl or aroyl, a carboxylic acid ester group, unsubstituted or substituted carbamoyl or sulfamoyl, alkylsulfonyl, arylsulfonyl or alkylsulfinyl,

$R^1$ and $R^2$ may also, together with the methine group, be a cyclic radical and $R^2$ may also be aryl or hetaryl,

$R^3$ and $R^4$ are hydrogen, unsubstituted or substituted alkyl, aryl, hetaryl, a carboxylic acid ester group, alkanoyl or aroyl and

$R^3$ and $R^4$ may also together be a cyclic radical, *characterised in that* a compound of the formula II

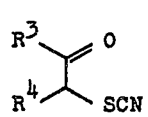

II

is reacted with a compound of the formula III

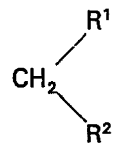

III

in the presence of a base.

2. A process as claimed in claim 1, *characterised in that* a compound of the formula

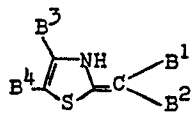

Ia

where

$B^1$ and $B^2$ are cyano, $C_1$—$C_4$-alkoxycarbonyl, $C_1$—$C_4$-alkanoyl, substituted carbamyl or hetaryl and $B^3$ and $B^4$ are unsubstituted or chlorine-, bromine-, $C_1$—$C_4$-alkoxy-, methyl-, acetylamino- or $C_1$—$C_4$-alkylmercapto-substituted phenyl, methyl or ethyl, is prepared.